# EUROPEAN PATENT SPECIFICATION

(11) **EP 3 107 507 B1**
(45) Date of publication and mention of the grant of the patent: **11.11.2020**
(21) Application number: 15711558.5
(22) Date of filing: 09.02.2015
(51) Int. Cl.: A61L 15/12, A61L 15/14, A61F 5/01, A61F 2/78, A61F 5/058, B33Y 10/00, B33Y 80/00

(54) **THERMOPLASTIC ARTICLE**
THERMOPLASTISCHER ARTIKEL
ARTICLE THERMOPLASTIQUE

(30) Priority: 21.02.2014 GB 201403054; 28.11.2014 GB 201421189
(43) Date of publication of application: 28.12.2016
(73) Proprietor: Torc2 Ltd., Leamington Spa, Warwickshire CV32 4RA (GB)
(72) Inventor: TAYLOR, Ronald Vincent, Leamington Spa Warwickshire CV32 4RA (GB); HOLLAND, Sandra, Leamington Spa Warwickshire CV32 4RA (GB)
(74) Representative: Dean, Alexander Bruce
(86) International application number: PCT/GB2015/050340
(87) International publication number: WO 2015/124900

(56) References cited:
- EP-A1- 0 338 815
- EP-A2- 2 671 544
- WO-A1-00/57821
- GB-A- 1 406 723
- GB-A- 2 513 536

## Description

### Field

The invention relates to a thermoplastic article, in particular for orthopaedic application.

### Background

Orthopaedic supports, for instance plaster casts for supporting a broken limb during healing, or supports for the long term rectification of limb abnormality are well known. Traditionally made from plaster of Paris, and now often from knitted fiberglass, epoxies or polyurethanes; such casts have the disadvantage of being of single use, and unpleasant to remove.

Having a single use cast can be a problem because for the majority of applications of such casts, more than one cast is required before the limb is healed or the abnormality is cured. It is therefore inefficient and wasteful to use multiple casts for a single treatment. For instance, during the healing of many fractures, a first cast is applied soon after the break. However, once the initial swelling subsides, the original cast becomes loose, and so must be replaced with a second cast, which is intended to remain until the fracture is healed. However, in some cases, when this cast is removed, healing is not complete and a third cast must be applied.

Further, the removal of the cast can be uncomfortable, or even painful, with burns from the cutting tools common, and can be traumatic for young children, who may not fully understand the process.

When used in the treatment of limb abnormalities, the "casts" are generally referred to as orthopaedic splint orthoses (OSOs). The problem of cast replacement is exacerbated in such treatments, as the OSOs are generally in place for relatively long treatment periods and need to be periodically replaced or adjusted to allow for incremental positional changes and/or patient growth. For instance, in the treatment of hip dysplasia the patient will often be anaesthetised to prevent the hips moving out of the manipulated position, whilst a plaster splint is applied and allowed to set hard enough to prevent movement. Application of such casts is therefore a surgical procedure. Typically the treatment duration is 6 months, although it can be far longer, and during this time the OSO must be adjusted/replaced to allow for the incremental change in hip configuration, and as the patient will often be an infant, to allow for patient growth. This adjustment typically occurs every 6-8 weeks, and can only be achieved by cutting off the old cast and replacing with a new plaster cast. As the patient will often be anaesthetised, this can be traumatic for the patient, in particular for infants with no understanding of why they are being treated in this way.

In the treatment of foot misalignment, as a further example, a splinting device known as an Ankle Foot Orthosis (AFO) is used. This device is produced in a thermoplastic material by an Orthotics Technologist from a plaster mould of the patients lower leg/ankle/foot made under the supervision of the attending clinician. The position of the foot is then incrementally adjusted at regular intervals until the desired position is achieved. To carry out the adjustment to the AFO it must be removed, sent back to the Orthotics Workshop where a skilled technologist heats up the thermoplastic and manipulates the shape to hold the foot in the new position as determined by the clinician. This adjustment often takes several days resulting in a delay before the AFO can be reapplied and requiring a repeat visit to the hospital or clinic.

Orthopaedic supports for prosthetic limbs have also been known for many years, and the interface between the limb and the prosthetic often includes a cushioned socket in which the amputees stump is located. The degree of comfort is heavily dependent on the fit of the stump in the socket and poor fit results in painful rubbing and pressure sores.

WO 00/57821 describes a splinting device comprising a flexible envelope which is filled with a thermoplastic material that has a solid-liquid phase change temperature in the range of 40 to 60°C. The envelope also comprises an electrical heating device contained within the envelope.

The invention is intended to overcome or ameliorate at least some aspects of the above problems.

### Summary

The scope of this invention is defined by the claims. Any references in the description to methods of treatment refer to the products of the present invention for use in a method for treatment of the human (or animal) body by therapy (or for diagnosis).

Accordingly, in a first aspect of the invention there is provided a thermoplastic article for orthopaedic application comprising a substrate, the substrate including in the range 57 to 95 wt% of a thermoplastic polymer and in the range 5 to 30 wt% of a wax; wherein, the article is plastic at a temperature in the range 40°C to 60°C; the article further comprising a flexible heating unit; and wherein the article is at least partially of a cellular construction, comprising cells, such that heat can be applied to selected cells. As used herein the term "plastic" is intended to mean "easily shaped or moulded", and is not limited to plastics materials.

The combination of the thermoplastic polymer and wax is generally homogeneous, the wax generally being dissolved in the polymer and so preventing separation from occurring. This provides for a substrate which can be reversibly softened and hardened between a rigid and a mouldable (often gelatinous) material at temperatures in the range 40°C to 60°C. It is an advantage of the invention that the mixture of thermoplastic and wax remains homogeneous throughout the multiple softening-hardening cycles encountered during the lifetime of the article.

The upper limit for the plastic temperature range is selected to ensure that the mouldability/plasticity is induced at temperatures which are easy to achieve through gentle heating, and such that the article will not cause discomfort or burning during application and moulding around the limb. The lower limit is such that the article will be rigid at room and body temperature, and will not begin to soften under normal wearing conditions (such as brief immersion in warm water, or showering). Often the upper limit will be 59°C, or 57°C, and is generally above 55°C. Reducing the upper limit of the mouldability range reduces the energy required to convert the article to its mouldable state, however, it has been found that below 55°C it can become difficult to mould the article by hand. Often the lower limit will be around 39°C or 40°C, often around 45°C, or 50 °C, rising as high as 55 °C in some cases. Increasing the lower limit helps to ensure that accidental softening of the article does not occur during day-to-day activities.

When used in orthopaedics, inducing mouldability in these ranges allows the clinician to apply the article to the limb in the plastic state and cool the article within minutes to form a rigid cast, OSO or similar structure. The fit can then be checked and readjusted if necessary, all within the time frame of a single visit. Further, the article can be softened and removed easily, by simple heating of the substrate with the application of only low levels of heat, such that there is no risk of discomfort or burning to the limb. Another advantage of the article of the invention is that where complete removal is not required, for instance because only a minor adjustment is needed, this can be achieved through the application of heat to key sections of the article, such that only these sections become plastic and mouldable, the remaining sections of the article being untouched. This dramatically improves the flexibility of use.

Further, the article itself is not damaged in the removal process, allowing for reuse. Where reuse is for a new wearer, the article can be fully cleaned and sterilised prior to this. As such, the article of the invention provides for the gentle removal and reuse of a cast or OSO (whether with a single patient, or after sterilisation with another patient), reducing stress to the patient, and reducing general wastage. Further, the ability to soften only some regions of the article means that in many cases it need not even be removed. For instance, in the case where a cast is being resized to allow for a reduction in post fracture swelling, only the part of the cast closest to the fracture need be softened, and manipulated. Similarly, where the cast wearer is an infant with hip dysplasia, traditional Spica casts (casts used to hold the legs in an open fixed position usually with a bar between the legs to maintain the splayed arrangement ensuring the correct alignment of ball and socket hip joint whilst the joint heals and matures) must be removed every 6-8 weeks, by sawing or cutting, to allow for growth of the infant and so avoid pressure sores and loss of circulation caused through cast tightening. This process of removal and replacement is often done under anaesthetic to avoid violent leg movements which could pull the hip out of alignment and which often occur due to the fear and pain associated with cast removal. Where such casts are substituted for the articles described, shape changes can be made through simple heating of the appropriate sections of the article, allowing for loosening and shape adjustment as appropriate. Further, as the process requires only the application of gentle heat, these changes can be carried out in a non-surgical (clinic) environment in a short space of time. This dramatically reduces the costs, risk and inconvenience of such treatments. Further, for many types of OSO, there is a delay in treatment where once the OSO is removed, it must be analysed by a clinician and sent away for specialist adjustment by an orthotist. This delay in treatment can be avoided through use of the article of the invention, as adjustment can happen at the clinic, with just one patient visit. This considerably improves the patient experience.

Further, in comparison to known casts and OSO's the article is lightweight to wear and comfortable, as it has been moulded to accurately fit the wearers physical shape. It protects the limb by providing impact dampening so that the inevitable knocks to the limb are less painful than with known casts, and is water resistant allowing for warm baths or showers to be taken by the wearer, and permitting swimming where desired.

To facilitate the softening and adjustment of selected parts of the article, the article comprises cells. Specifically, the article is of at least partially cellular construction, comprising (for instance) pouches of the substrate, such that heat can be applied to just selected cells, inducing softening and hence mouldability in these cells only. The cells may be individually constructed such that they can be assembled in a range of configurations to form the article. The assembly configuration would depend upon the specific intended use (limb and condition) of the article.

The thermoplastic polymer may be any polymer but will often be selected from methacrylates (for instance PMMA), polyamides, polyethylenes, polypropylenes and poly acetates (for instance polyvinyl acetate or ethylene vinyl acetate), or copolymers thereof. In many cases the thermoplastic polymer will comprise ethylene vinyl acetate (EVA) as this polymer has good physical properties, in particular with regard to softening points. The thermoplastic polymer is present in the substrate in the range 57 wt% to 95 wt% of the substrate, often in the range 60 wt% to 90 wt% of the substrate, often 70wt% to 80 wt% of the substrate. Outside these ranges it has been found that the substrate may not soften in the desired temperature range. In many cases, where the thermoplastic polymer is an acetate, such as EVA, the acetate content of the copolymer will be in the range 15 to 40 wt% of the copolymer, as at these relative vinyl:acetate proportions, the best substrate stability can be obtained.

The wax may be a natural or a synthetic wax, often the wax will be an animal, vegetable or petroleum wax, for instance, paraffin wax. Often the wax will be a vegetable or petroleum wax, as some sectors of society disapprove of the use of animal products. Often the wax will comprise paraffin wax due to the ready availability of this wax, and the range of melting points available. Often a paraffin wax with a melting point in the range 40°C to 55°C. The wax is present in the range 5 to 30 wt% of the substrate, often in the range 15 wt% to 25 wt%, or around 20 wt% of the substrate. The amount of wax present will depend upon the melting point of the thermoplastic polymer selected, such that higher melting point polymers, require a higher proportion of wax to induce moldability in the temperature range desired. Clearly, the melting point of the wax selected will also affect the level that must be present in the substrate. Often where the thermoplastic polymer is EVA, present in the range 57 wt% to 95 wt% of the substrate, the wax will be paraffin wax, present in the range 15 wt% to 25 wt% of the substrate.

The substrate may comprise first and second surfaces, and a coating on at least a part of one or both of the first and second surfaces. The presence of the coating allows for containment of the substrate, for instance into a cellular structure, and improves the resilience of the article by protecting the surface of the substrate. This can extend the life of the article, providing for more reuses of the article than would be the case if the coating were absent. Often the coating will substantially cover the surface of the first and/or second surface of the substrate. Often the coating will be present on the first and second surface, and where multiple surfaces are present, on most if not all of these. Such a configuration provides for substantially complete, or complete, surface coverage of the article with the coating. It will often be the case that the coating comprises polyurethane and/or polyethylene as when used in coatings these polymers are tough and flexible. However, silicone or other common medical coatings may also be used. Often the coating will comprise a film laminated onto at least one surface of the substrate, although the coating may be applied using any of a wide variety of known coating methods, including painting, vapour deposition, spray coating, roll coating and combinations thereof.

It is an advantage of the article that, unlike known casts and orthotics, it is fully washable. As such, the wearer can wash and shower (in warm water), and keep the article itself clean. This improves both hygiene and wearer morale.

The substrate may further comprise an additive selected from fibres, fillers, antimicrobials, flame retardants, heat transfer agents, colourants, and combinations thereof. The choice of additive depends upon the precise requirements of the article in its intended use.

The fibres are typically present to improve rigidity and flexural strength of the substrate and hence the article. They can be selected from polymeric fibres, natural fibres, carbon fibres and combinations thereof. For instance, a polymeric fibre may be selected from polypropylene or other thermoplastic fibre, such fibres generally increase flexural strength. Natural fibres would include fibres such as flax, jute, cotton, hemp, coir, bamboo and combinations thereof. Typically the natural fibres for use in the invention will be of plant origin, to avoid insult to those who disapprove of the use of animal products. Natural fibres can add bulk to the substrate and increase rigidity. Where present, the polymeric fibre may be in the range 5 wt% to 20 wt% of the substrate, natural fibres may also be present at these levels. Carbon fibres may be used, to impart stiffness and flexural strength to the substrate. Where present, carbon fibres are typically found at levels in the range 2 wt% to 8 wt%, as their low density allows these to be used at low levels. Where a high level of stiffness is required, a carbon fibre mat could be embedded into the substrate.

Fillers as used herein will typically be mineral fillers, such as magnesium silicate, calcium carbonate, kaolinite, barites and combinations thereof. They are typically added to increase the rigidity of the article when in its solid form, although they may also be added to reduce tackiness of the substrate at manufacturing processing temperatures, if the particular thermoplastic polymer and wax combination selected becomes difficult to process. The fillers have been found to most effectively increase the rigidity of the article when present at levels in the range 5 wt% to 25 wt% of the substrate.

Antimicrobials and/or antivirals may also be used, to reduce viral and/or microbial contamination of the article, and transfer of such contamination to the skin, in turn reducing infection and improving cleanliness. Where present, the antimicrobial and/or antiviral will typically be present in the range 1 wt% to 2 wt% of the substrate.

Flame retardant additives may also be present, to reduce the combustibility of the substrate and hence the article. Typically the flame retardant will be non-halogenated as it is undesirable to contact halogenated compounds with the skin. Often it will be an organo phosphorous compound, for instance, the flame retardant additive may be selected from triphenyl phosphate (TPP), ammonium polyphosphate, resorcinol bis(diphenylphosphate) (RDP), bisphenol A diphenyl phosphate (BADP), tricresyl phosphate (TCP); dimethyl methylphosphonate (DMMP); aluminum diethyl phosphinate and combinations thereof. Often ammonium polyphosphate will be used as this flame retardant has good compatibility with the substrate components. Often the flame retardant additive will be present at a level in the range 20 wt% to 35 wt% of the substrate as at these levels the retardancy levels set out in the UL-94 V0 plastics safety test can be fulfilled.

Heat transfer agents may be present, to improve the conductivity of heat through the substrate, ensuring rapid and even heating/cooling of the article, particularly when induction or conduction methods are used. Often the heat transfer agent will be metallic, often a metal powder or fibre. Often the metal will be steel or aluminium as powders/fibres of these metals are readily available as low cost. Often the metal will be present in the range 5wt% to 10 wt% of the substrate.

Colourants may also be added to the substrate and/or coating. The use of pigments and/or dyes as colourants can improve the appearance of the article, making it more desirable, for instance to children, thereby improving patient compliance where, for instance, the article is a OSO which can be removed (such as ankle-foot orthoses (AFOs) used to make correctional changes to an individual's gait). It can also acts as a means of coding the article, so that the substrate used, intended use of the article can be readily recognised.

The article further comprises a flexible heating unit which will often be at least partially embedded within the substrate. Often the heating unit will be fully embedded within the substrate. Embedding may be through fully or partially sandwiching the heating unit between layers of substrate (for instance by lamination) or by immersion of the heating unit (or other embedded component) into the substrate when the substrate is in a flowable state.

The heating unit will typically be an electrical heating unit, however, for specialist applications (for instance where it is desirable that the article be invisible to X-rays or radio waves, so that the limb may be viewed without the need to remove the article), the heating unit may comprise capillary mat technology, so that hot water is used to soften the article for remoulding or removal.

Passing heat through the unit ensures rapid even heating of the article, in particular of the substrate where the heating unit is embedded within this, such that moulding of the article can begin without undue delay. The heating unit may be a thin film heater, a silicone heater, metallic mesh heating elements, induction coils, capillary matting or combinations thereof. The heating unit may be formed by printing, in particular by 3D printing to form a printed circuit. The use of printed circuits allows for rapid production of the unit and decentralised manufacture of the article where necessary. With the exception of capillary mat systems, each of the above heating units may be heated using electrical power, for instance via connectors (such as, flat multi-pin connectors) for use with an external power supply, such that the connectors and heating unit form a circuit with the external power supply. The external power supply may be DC (such as a PV cell or battery) or AC (such as mains power which would be fed into the circuit via an AC-DC adaptor). Where a DC power source is present, this may be incorporated into the article. In addition, where mesh heating elements or induction coils are used, oven heating can be effective in activating the heating unit (off the limb), such that the article is becomes plastic more rapidly than if the heating unit were absent.

Often the article will comprise a textile cover, improving the comfort of the article against the skin, and providing for a replaceable, washable, element to the article; for instance, washing and replacement can occur when the article is removed for adjustment. The cover may be woven or non-woven and of single or multiple layers. The textile cover may be designed to lie next to the skin of the wearer in use, or to encase the outer surface of the article, or both. Where the cover lies next to the skin, comfort can be improved, when it encases the outer surface of the article, it can provide a decorative and protective function. Both functions may be fulfilled when the cover is on both the inner surface and the outer surface of the article in use. A key benefit of including a textile cover is that this may be replaced entirely between applications of the article to different wearers. Such that, for each wearer, the article appears to be brand new, relieving any concerns about hygiene that may otherwise be present. Whether or not the textile cover is replaced between wearers, it will often include an antimicrobial finish to improve hygiene.

The textile cover may include one or more shape retainers. These may be to help hold the article in position during cooling and setting, or to hold the article in position where it is of a design such that a limb is not entirely encased, or both. Often the shape retainers will be compression straps. The compression straps may include cinchable hook- and-loop fastenings, double loop buckle straps or similar.

As an addition or alternative to a textile cover for certain applications a metallic protective cover, for instance a chain mail cover, may be present. This can be advantageous where the article is being used in veterinary applications, to prevent the animal from damaging the article through, for instance, biting.

The article may further comprise protectors, in some instances as part of the textile cover, in some instances not. As used herein the term "protector" is intended to be any component other than the coating, or textile cover (described separately) which performs the function of protecting the structural integrity of the article. For instance, a protector may be a sole applied to a surface of the article where it is intended that the surface contact the ground during walking (such as with AFOs), thereby preventing wear to the article, helping to keep the article clean and providing a more secure contact with the ground than would be provided using the article alone. The presence of the protector may therefore allow the wearer of the article to walk safely. Alternatively, the protector may be a moulded slipper placed around the part of the article protecting the foot.

The article may further comprise one or more of a range of electronic inserts selected from, thermostats, pressure sensors, temperature sensors, blood flow sensors, skin and/or muscle stimulators, output chips, or combinations thereof. These inserts can provide for inter-visit monitoring of the wearer, providing an early warning of problems with their health or usage of the article. The inserts may be provided on the surface of the article, or embedded therein, typically they will be embedded in the article, as embedding provides protection for the sensor from accidental damage and tampering. Often embedding will be in the substrate itself.

Thermostats may be present to provide temperature control to the article, either triggering heating/cooling where the article temperature moves outside defined parameters, or causing an alarm, such that the wearer can arrange for heating or cooling of the article.

Pressure sensors may also be included, and can be of particular use where the wearer is a small child or infant who cannot alert those around to the specifics of their discomfort. Where pressure is observed to be above a given value, this can be an early warning that pressure sores will form, and can alert either the wearer or clinician that adjustment of the article is required. For some uses of the article this can be entirely expected, for instance, where the article is being used in the treatment of hip dysplasia in infants, the infant is expected to grow and remoulding will be required several times before treatment is complete. In such cases, the indicators provided by a pressure sensor could be invaluable in providing guidance as to the timing of the next adjustment of the article. Alternatively, pressure applied to the sensor during wear can be used to monitor not only the pressure itself, but the duration of wear. For instance, the data from a pressure sensor can be used as an indicator of wearer compliance, in cases where the article is removable and should be worn for a defined period of every day.

Temperature sensors can also be provided, these can supply information as to the general health of the wearer, for instance, elevated temperature readings could be indicative of fever and fever of infection. Temperature sensors can also provide an indicator that the article has been brought into contact with a heat source such that it may have become entirely or partially deformed and needs to be remoulded.

Blood flow monitors may also be included, and can be of use as poor blood flow could be indicative that the article is (or has become) too tight, constricting blood flow and possibly causing damage to the limb. For instance, blood flow monitors can be used in the treatment of hip dysplasia as constriction of blood flow could be indicative of infant growth and the need to adjust the article. In addition, it may be that to induce some changes in bone structure (such as to remove club feet) a limited amount of blood flow constriction is unavoidable, and in these situations a blood flow monitor can be of use in ensuring that constriction is at acceptable levels, and to monitor the improvement in blood flow as the limb slowly changes shape.

Skin and/or muscle stimulators may be present. These can help to increase circulation, where for instance in order to induce a change in bone structure it is necessary to inhibit blood flow. Such stimulators can also assist in the healing and/or maintenance of the skin and muscle tone close to the site of injury during healing.

Where sensors are present, these can provide direct feedback to the wearer of the article whether in terms of numerical read outs forming part of the article, or audible alarms when parameters fall outside the defined norms. However, it can be useful in many cases for the sensor read outs to be transmitted from the article either to a local recorder (such as a mobile device, laptop or PC), or to the clinician. As such, it can be beneficial to include RF chips for wireless communication of sensor data to an external recorder.

The electronic inserts will generally be printed and form part of a circuit also including a heating unit (where present). Generally the electronic inserts will require power, for instance from a battery of PV cell, each of which could be part of the article. Often, where such power sources are present, they will also be capable of powering the heating unit; although in many cases the power required to induce softening of the article will be supplied externally.

Manufacture of the article of the first aspect of the invention may therefore comprise, in a second aspect of the invention, the steps of:
dispersing a wax in a thermoplastic polymer to form a substrate;
providing a flexible heating unit;
cooling the substrate to mouldable temperatures; and
moulding to form an article having an at least partially cellular structure.

Optionally, the dispersal step may be carried out at an elevated temperature, to facilitate mixing of the thermoplastic polymer and wax. In many cases, mixing will continue until a homogenous mixture is obtained.

The substrate may be fully or partially coated to improve the durability of the article and a textile cover may be applied.

Further, additives selected from fibres, fillers, antimicrobials, flame retardants, heat transfer agents, colourants and combinations thereof may be added to the thermoplastic polymer and wax dispersion during substrate formation, or subsequent to this step, but prior to cooling.

As explained above, existing techniques for preparing orthopaedic supports such as splinting devices for patients typically involve labour and machine intensive manufacturing procedures at specialist orthopaedic workshops. The delays associated with constructing, repairing and returning equipment to patients is often very long, typically taking several weeks. The inventors of the present invention have found that 3D printing techniques are particularly effective at manufacturing the articles of the invention (such as the splinting devices mentioned above) from the materials described herein.

The 3D printing processes typically comprise the steps of analysing the size and shape of the body or part of the body of a patient; using the resulting information to identify an optimum architecture and construction for an article as described herein (e.g. a splinting device); and fabricating an article to meet these specifications using 3D printing. It is often the case that the analysing step is performed using 3D imaging techniques to map a patient's body shape or shape of a specific area. This in turn may be used to generate a virtual model of a medical device to optimally treat a patient's particular condition with a particular body shape. Modifications can be included, typically from medical professionals, which deviate from the generated virtual image based on specific expertise. The resulting information can be converted into instructions for a 3D printing system. This acts as a template which can be read by a 3D printer in order to create the well-fitting article of the invention.

Not in accordance with the present invention, there is also provided a computer readable medium which when executed on a processor causes the computer to perform the steps of: (a) generating a first output indicative of the size and shape of a patient's body or part thereof based on an input containing information regarding the dimensions of a patient's body or part thereof; (b) determining the shape and size of an article according to the first aspect of the invention adapted for treating the patient based on the first output; and (c) generating instructions for a 3D printer in order for said printer to construct the article according to step (b).

The term "3D printing" is intended to cover processes which involve the successive build-up of layers of material in order to create a three dimensional structure. Typically, such techniques are performed by computers. This term is not intended to be limited to specific types of 3D printing e.g. wherein the nozzle remains stationary and the base of the printer from which fabrication occurs moves or where the base is immoveable and the nozzle moves in three dimensions. It is possible in some situations for a support structure to be used as a framework or scaffold in order to assist the manufacture of the article. This scaffold or framework can be broken off or dissolved after the article has been fully formed. Typically, water soluble scaffold materials are used.

A significant advantage of this technique over existing techniques is that the turnaround time for a patient to receive his or her article is greatly reduced and the need for specialist workshops and specialised craftsman is minimised. This benefit is especially pronounced for young patients whose bodies grow and change quickly.

The orthopaedic application may be selected from human or animal application and may be orthotics, bone or muscle healing, prosthetics or combinations thereof. In a third aspect of the invention there is provided the use of an article according to the first aspect of the invention in prosthetics. The article of the invention can be of particular use in these fields as they are well known for the inadequacy of treatment in terms of materials wastage and discomfort to the wearer during healing/treatment and removal of the cast, or the wearing of prosthetics.

As used herein the term "orthotics" is intended to include any application of the article in orthoses, for correcting the muscular and/or skeletal system. In particular, the orthotics may comprise a use selected from Spica casts, Ponsetti casts, splinting and AFOs.

Spica casts are described in detail above, and are used in the treatment of hip dysplasia, in particular in infants. Ponsetti casts are used for the treatment of club foot by repeated casting the foot by stretching the ligaments and other soft tissue until the deformity is removed. AFOs are ankle to foot splint type devices that are used to make positional corrections to a patient's gait, as described above. There are many conditions that affect the way in which a patient walks and this type of device is used in large numbers. The extent of the correction needed determines both the length of time the OSO must be worn and the number of times the OSO must be altered to create a new positional change or to reduce chafing caused by incorrect fit adjustment (which occurs in 25-30% of fittings). When the article is used as an AFO, an initial basic shape is produced, which can then be softened by heat to adjust both the fit to the patient's limb and those areas around and below the foot that are used to change the distribution of weight on the foot to correct the gait. There are also a variety of splinting applications included within this definition of orthotics. For instance, limb correction splinting associated with cerebral palsy sufferers where the corrections involve incremental positional changes and where the current splinting system entails re-modelling at a remote specialist orthotic centre are a useful application of the articles of the invention. The current method of treatment requires two clinic visits at each stage, between which the splint is remodelled as a result of limb change from the initial period of correction. This results in delays in treatment, where because the splint is not being worn, no progress is being made. Further, when the remodelled splint is received, 25% of the time it is incorrect, and the fit must be changed, causing further delay and further visits to the clinic. The article of the invention can be remodelled at the clinic, while the wearer waits, entirely avoiding the treatment delay while the splint is remodelled at the workshop. The correction of idiopathic toe walking also requires serial recasting, and so the use of the article in the correction of this deformity could also be beneficial.

Bone or muscle healing as used herein encompasses the use of the article as a replacement for "traditional" casts, applied when bones are fractured, to stabilise the limb during bone healing. One advantage of the article is that it can be heated and reset, where for instance it is a joint such as an ankle or wrist which had been broken, and periodic manipulation is required to ensure that full motion is retained after removal of the article/cast.

The term "prosthetics" is intended to include the use in improving the comfort of any prosthetic, in particular the article can be used in prosthetic limb sockets, as a socket liner. The liner may be softened and moulded to the shape of the limb stump, ensuring a good fit, and hence a reduction in chafing and sores resulting from rubbing of the limb in the prosthetic socket. In some cases, the socket liner could be formed from an article comprising two substrates each with different softening temperatures. A lower temperature softening material being in contact with the limb, this lower temperature material possibly even having a softening temperature below 37°C, so that it is mouldable and can move with the limb end in use. The substrate with the higher softening temperature being in contact with the prosthetic. Alternatively, the lower temperature substrate may be capable of being moulded within the temperature range 40°C to 60°C so that the substrate is rigid at body temperature, forming a stable socket which accurately reproduces the shape of the stump. This can be achieved either in an clinic without having to send the prosthetic away for specialist shaping or even by the amputee at home whenever a change in fit comfort is required.

The use may comprise the steps of:
heating the article having an at least partially cellular structure to a temperature in the range 40°C to 60°C using the flexible heating unit;
forming the article around a limb; and
cooling the article to a temperature in the range 15°C to 39°C, to provide a rigid support for the limb; wherein said use is not a method of medical treatment. A further step comprising reheating the whole or part of the article to a temperature in the range 40°C to 60°C, and remoulding the article around the limb, or removing the article from the limb may also be present.

Cooling is typically carried out by a method selected from, gradual loss of heat to environment, application of cold packs, immersion in cool water, or combinations thereof.

The use may further comprise activating one or more electronic inserts in the article.

The invention therefore provides a cellular thermoplastic article for orthopaedic application comprising:
a substrate comprising in the range 57 wt% to 90 wt% of EVA, in the range 10 wt% of paraffin wax,
and optionally an additive selected from fibres, fillers, antimicrobials, flame retardants, heat transfer agents, colourants, and combinations thereof;
a flexible heating unit embedded in the substrate;
optionally an electronic insert selected from, thermostats, pressure sensors, temperature sensors, blood flow sensors, skin and/or muscle stimulators, output chips, or combinations thereof;
optionally a coating optionally comprising polyurethane and/or polyethylene laminated onto at least one surface of a substrate;
optionally a woven textile cover optionally including shape retainers and/or protectors;
wherein, the article is plastic at a temperature in the range 50°C to 59°C, and the article is at least partially of a cellular construction, comprising cells, such that heat can be applied to selected cells, and the orthopaedic application is optionally selected from orthotics, bone or muscle healing, prosthetics or combinations thereof.

### Brief Description of the Drawings

In order that the invention may be more readily understood, it will be described further with reference to Figure 1 and to the specific example hereinafter.
Figure 1 is a schematic cross-sectional diagram through an article of the invention.

### Detailed Description

Figure 1 shows a part of a thermoplastic article 5, showing in cross-sectional view a substrate 10 comprising the following components:
58 wt% EVA
18 wt% ammonium polyphosphate
14 wt% paraffin wax
7 wt% magnesium silicate
2.3 wt% jute
0.7 wt% colour

A polyurethane film 15 is laminated to both surfaces 20 of the substrate, and a printed circuit heating unit 25 embedded within the substrate by lamination of two layers of substrate 10 to either side of the heating unit 25 such that it is sandwiched within. Also embedded within the substrate 10 is a temperature sensor 30. A textile coating 35 surrounds the polyurethane film coated substrate 10.

The article 5 can be applied directly to a limb to control movement.

### Example

The article described with reference to Figure 1 above is mouldable by hand (plastic) at temperatures from 39-40°C to 59-60°C, is fully washable and conforms to the UL-94 fire retardancy requirements. It is therefore a suitable alternative to known cast materials.

It should be appreciated that the article, method and use of the invention are capable of being implemented in a variety of ways, only a few of which have been illustrated and described above.

## Claims

1. A thermoplastic article for orthopaedic application comprising a substrate, the substrate including in the range 57 to 95 wt% of a thermoplastic polymer and in the range 5 to 30 wt% of a wax; wherein, the article is plastic at a temperature in the range 40°C to 60°C; the article further comprising a flexible heating unit; and **characterised in that** the article is at least partially of a cellular construction, comprising cells, such that heat can be applied to selected cells.

2. An article according to claim 1, wherein the substrate comprises first and second surfaces, and a coating on one or both of the first and second surfaces.

3. An article according to claim 1 or claim 2, wherein the substrate further comprises an additive selected from fibres, fillers, antimicrobials, flame retardants, heat transfer agents, colourants and combinations thereof.

4. An article according to any of claims 1 to 3, wherein the cellular construction comprises pouches of substrate.

5. An article according to any preceding claim, wherein the heating unit is a circuit comprising connectors for use with an external power supply.

6. An article according to any preceding claim, further comprising a textile cover.

7. An article according to any preceding claim, wherein the article further comprises protectors; or wherein the article further comprises an electronic insert selected from, thermostats, pressure sensors, temperature sensors, blood flow sensors, skin and/or muscle stimulators, output chips, or combinations thereof.

8. An article according to any preceding claim, wherein the orthopaedic application is selected from orthotics, bone or muscle healing, prosthetics or combinations thereof.

9. Method of making an article according to any preceding claim, comprising the steps of:
dispersing a wax in a thermoplastic polymer to form a substrate;
providing a flexible heating unit in the substrate;
cooling the substrate to mouldable temperatures; and
moulding to form an article having an at least partially cellular structure.

10. A method according to claim 9, further comprising adding an additive selected from fibres, fillers, antimicrobials, flame retardants, heat transfer agents, colourants and combinations thereof to the thermoplastic polymer and wax dispersion either during substrate formation, or subsequent to substrate formation, but prior to cooling.

11. A method according to claim 9 or claim 10, wherein the article is made using 3D printing.

12. Use of an article according to any of claims 1 to 8 in prosthetics, comprising the steps of:
heating the article having an at least partially cellular structure to a temperature in the range 40°C to 60°C using the flexible heating unit;
forming the article around a limb; and
cooling the article to a temperature in the range 15°C to 39°C, to provide a rigid support for the limb; wherein said use is not a method of medical treatment.

13. Use according to claim 12 further comprising activating one or more electronic inserts in the article.

## Patentansprüche

1. Thermoplastischer Gegenstand zur orthopädischen Anwendung, umfassend ein Substrat, wobei das Substrat im Bereich von 57 bis 95 Gew.-% eines thermoplastischen Polymers und im Bereich von 5 bis 30 Gew.-% eines Wachses enthält, wobei der Gegenstand bei einer Temperatur im Bereich von 40°C bis 60°C formbar ist, wobei der Gegenstand ferner eine flexible Heizeinheit umfasst, **dadurch gekennzeichnet, dass** der Gegenstand zumindest teilweise aus einem zellulären Aufbau besteht, der Zellen umfasst, so dass Wärme auf ausgewählte Zellen übertragen werden kann.

2. Gegenstand nach Anspruch 1, wobei das Substrat eine erste und eine zweite Oberfläche und eine Beschichtung auf einer oder beiden der ersten und der zweiten Oberflächen umfasst.

3. Gegenstand nach Anspruch 1 oder Anspruch 2, wobei das Substrat ferner ein Additiv, ausgewählt aus Fasern, Füllstoffen, antimikrobiellen Mitteln, Flammschutzmitteln, Wärmeübertragungsmitteln, Farbmitteln und Kombinationen davon umfasst.

4. Gegenstand nach einem der Ansprüche 1 bis 3, wobei der zelluläre Aufbau Beutel aus Substrat umfasst.

5. Gegenstand nach einem der vorhergehenden Ansprüche, wobei die Heizeinheit eine Schaltung mit Anschlüssen zur Verwendung mit einer externen Stromversorgung ist.

6. Gegenstand nach einem der vorhergehenden Ansprüche, der ferner einen Textilbezug umfasst.

7. Gegenstand nach einem der vorhergehenden Ansprüche, wobei der Gegenstand ferner Protektoren umfasst, oder wobei der Gegenstand ferner einen elektronischen Einsatz umfasst, der ausgewählt ist aus Thermostaten, Drucksensoren, Temperatursensoren, Blutstromsensoren, Haut- und/oder Muskelstimulatoren, Ausgabechips oder Kombinationen davon.

8. Gegenstand nach einem der vorstehenden Ansprüche, wobei die orthopädische Anwendung ausgewählt ist aus Orthotik, Knochen- oder Muskelheilung, Prothetik oder Kombinationen davon.

9. Verfahren zur Herstellung eines Gegenstands nach einem beliebigen vorhergehenden Anspruch, umfassend die Schritte:
Dispergieren eines Wachses in einem thermoplastischen Polymer zur Bildung eines Substrats,
Bereitstellen einer flexiblen Heizeinheit in dem Substrat,
Abkühlen des Substrats auf formbare Temperaturen, und
Formen zur Bildung eines Gegenstands mit einer zumindest teilweise zellulären Struktur.

10. Verfahren nach Anspruch 9, ferner umfassend die Zugabe eines Additivs, ausgewählt aus Fasern, Füllstoffen, antimikrobiellen Mitteln, Flammschutzmitteln, Wärmeübertragungsmitteln, Farbmitteln und Kombinationen davon, zu der thermoplastischen Polymer- und Wachsdispersion entweder während der Substratbildung oder nach der Substratbildung, jedoch vor dem Abkühlen.

11. Verfahren nach Anspruch 9 oder Anspruch 10, wobei der Gegenstand unter Anwendung des 3D-Druckens hergestellt wird.

12. Verwendung eines Gegenstands nach einem der Ansprüche 1 bis 8 in der Prothetik, umfassend die Schritte:
Erhitzen des Gegenstands mit einer zumindest teilweise zellulären Struktur auf eine Temperatur im Bereich von 40°C bis 60°C unter Verwendung der flexiblen Heizeinheit,
Formen des Gegenstands um eine Gliedmaße, und
Kühlen des Gegenstands auf eine Temperatur im Bereich von 15°C bis 39°C, um eine starre Stütze für die Gliedmaße bereitzustellen, wobei diese Verwendung kein Verfahren der medizinischen Behandlung ist.

13. Verwendung nach Anspruch 12, ferner die Aktivierung eines oder mehrerer elektronischer Einsätze in dem Gegenstand umfassend.

## Revendications

1. Article thermoplastique pour une application orthopédique comprenant un substrat contenant un polymère thermoplastique dans une plage de 57-95 % pondéraux et de la cire dans la plage de 5-30% pondéraux,
- l'article étant à l'état plastique dans la plage de température de 40°C-60°C,
- l'article comprenant en outre une unité souple de chauffage, et article **caractérisé en ce qu'**il a
au moins en partie une construction cellulaire avec des cellules de façon que la chaleur puisse être appliquée à des cellules sélectionnées.

2. Article selon la revendication 1,
selon lequel
le substrat a une première et une seconde surface et un revêtement sur l'une ou sur les deux surfaces.

3. Article selon la revendication 1 ou la revendication 2,
selon lequel
le substrat comprend en outre un additif choisi parmi :
fibres, charges, anti-microbiens, retardants de flammes, agents thermo-conducteurs, colorants et leurs combinaisons.

4. Article selon l'une quelconque des revendications 1 à 3,
selon lequel
la construction cellulaire comprend des poches de substrat.

5. Article selon l'une quelconque des revendications précédentes,
selon lequel
l'unité de chauffage est un circuit comprenant des connecteurs pour être relié à une alimentation externe.

6. Article selon l'une quelconque des revendications précédentes, comprenant en outre une couverture textile.

7. Article selon l'une quelconque des revendications précédentes,
selon lequel l'article comprend en outre des protecteurs, ou un insert électronique choisi parmi : thermostats, capteurs de pression, capteurs de température, capteurs de débit sanguin, stimulateur de peau et/ou de muscles, puces de sortie ou leurs combinaisons.

8. Article selon l'une quelconque des revendications précédentes,
selon lequel
l'application orthopédique est choisie parmi orthèses, traitements des os ou des muscles, prothèses ou leurs combinaisons.

9. Procédé de fabrication d'un article selon l'une quelconque des revendications précédentes, comprenant les étapes consistant à :
- disperser une cire dans un polymère thermoplastique pour former un substrat,
- fournir une unité de chauffage souple dans le substrat,
- refroidir le substrat à des températures de moulage, et
- mouler pour former un article ayant au moins partiellement une structure cellulaire.

10. Procédé selon la revendication 9,
consistant en outre à :
- ajouter un additif choisi parmi : fibres, charges, anti-microbien, retardants de flammes, agent thermo-conducteurs, colorants et leurs combinaisons au polymère thermoplastique et à la dispersion de cire pendant la formation du substrat ou après la formation du substrat mais avant le refroidissement.

11. Procédé selon la revendication 9 ou la revendication 10,
selon lequel
on réalise l'article par une impression 3D.

12. Utilisation d'un article selon l'une quelconque des revendications 1 à 8, comme orthèses, comprenant les étapes suivantes consistant à :
- chauffer l'article ayant au moins partiellement une structure cellulaire à une température de l'ordre de 40°C-60°C en utilisant une unité de chauffage souple,
- former l'article autour d'un membre, et
- refroidir l'article à une température de l'ordre de 15°C-39°C pour obtenir un support rigide pour le membre, l'utilisation n'étant pas un procédé de traitement thérapeutique.

13. Utilisation selon la revendication 12, consistant en outre à activer un ou plusieurs inserts électroniques dans l'article.
